# EUROPÄISCHE PATENTSCHRIFT

(11) **EP 3 573 679 B1**
(45) Veröffentlichungstag und Bekanntmachung des Hinweises auf die Patenterteilung: **20.05.2026**
(21) Anmeldenummer: 18702207.4
(22) Anmeldetag: 25.01.2018
(51) Int. Cl.: A61M 1/14, A61M 1/36, A61M 39/20

(54) **NEUE BERUEHRSCHUTZVORRICHTUNG FUER MEDIZINISCHE FLUIDFUEHRENDE KASSETTE UND KASSETTE**
NOVEL CONTACT PROTECTION DEVICE FOR A MEDICAL FLUID-CARRYING CARTRIDGE, AND CARTRIDGE
DISPOSITIF DE PROTECTION CONTRE LES CONTACTS POUR CASSETTE CONDUISANT DES FLUIDES MÉDICAUX, ET CASSETTE

(30) Priorität: 30.01.2017 DE 102017101730
(43) Veröffentlichungstag der Anmeldung: 04.12.2019
(73) Patentinhaber: Fresenius Medical Care Deutschland GmbH, 61352 Bad Homburg (DE)
(72) Erfinder: LAUER, Martin, 66606 St. Wendel (DE)
(74) Vertreter: Bobbert & Partner Patentanwälte PartmbB
(86) Internationale Anmeldenummer: PCT/EP2018/051855
(87) Internationale Veröffentlichungsnummer: WO 2018/138208

(56) Entgegenhaltungen:
- DE-A1- 102011 108 781
- US-A1- 2008 093 246

## Beschreibung

Die vorliegende Erfindung betrifft eine medizinische fluidführende Kassette gemäß Anspruch 1. Ein Beispiel aus dem Stand der Technik ist in DE 10 2011 108 781 A1 offenbart.

Bei der Handhabung von fluidführenden Kassetten, beispielsweise von Blutkassetten, durch das medizinische Personal besteht stets die Gefahr, dass beim Auspacken einer Kassette aus der Sterilverpackung versehentlich Konnektionsstellen, welche während der Behandlung des Patienten zur Zugabe eines Fluids in die Kassette (kurz: Fluidzugabestellen der Kassette) genutzt werden, mit dem Finger oder einem Gegenstand berührt und somit kontaminiert werden. Aus der Praxis sind bereits verschiedene Lösungen hierfür bekannt, zum Beispiel in Form von lösbar verklebten Schutzfolien.

Eine Aufgabe der vorliegenden Erfindung ist es, eine weitere medizinische fluidführende Kassette vorzuschlagen.

Die erfindungsgemäße Aufgabe wird durch eine medizinische fluidführende Kassette mit den Merkmalen des Anspruchs 1 gelöst.

Die offenbarte Berührschutzvorrichtung zum Abdecken einer Konnektionsstelle einer, insbesondere medizinischen, fluidführenden Kassette für eine medizinische Fluidbehandlung weist wenigstens einen Abdeckabschnitt zum Abdecken der Konnektionsstelle vor Gebrauch der Kassette, d. h. bevor die Kassette an einer Fluidbehandlungsvorrichtung angebracht wird, auf. Sie weist ferner wenigstens einen ersten Verbindungsabschnitt, zum lösbaren Verbinden oder Halten der Berührschutzvorrichtung an der Kassette, auf.

Der wenigstens eine Abdeckabschnitt der Berührschutzvorrichtung ist auf oder über wenigstens einer Konnektionsstelle einer fluidführenden Kassette platzierbar und vorzugsweise breit genug, um diese Konnektionsstelle, oder mehrere Konnektionsstellen, der Kassette derart abzudecken, dass bei aufgesetzter Berührschutzvorrichtung bei üblichem Gebrauch kein Abschnitt der Konnektionsstelle durch Berührung mit den Fingern kontaminiert werden kann. Der Abdeckabschnitt kann alle dem Stand der Technik bekannten Formen haben, beispielsweise kann er plattenförmig sein. Er kann rechteckig, halbrund oder dergleichen ausgestaltet sein, usw. Die Größe des Abdeckabschnitts kann in Abhängigkeit zur Größe der im Einzelfall zur schützenden Konnektionsstelle ausgewählt sein.

Die Berührschutzvorrichtung weist eine Oberseite und eine Unterseite auf.

Die Erfindung betrifft eine medizinische, fluidführende Kassette mit wenigstens einer Berührschutzvorrichtung.

Bei allen folgenden Ausführungen ist der Gebrauch des Ausdrucks "kann sein" bzw. "kann haben" usw. synonym zu "ist vorzugsweise" bzw. "hat vorzugsweise" usw. zu verstehen und soll erfindungsgemäße Ausführungsformen erläutern.

Erfindungsgemäße Ausführungsformen sind ferner Gegenstand der Unteransprüche und Ausführungsformen.

Wann immer hierin Zahlenworte genannt werden, so versteht der Fachmann diese als Angabe einer zahlenmäßig unteren Grenze. Sofern dies zu keinem für den Fachmann erkennbaren Widerspruch führt, liest der Fachmann daher beispielsweise bei der Angabe "ein" oder "einem" stets "wenigstens ein" oder "wenigstens einem" mit. Dieses Verständnis ist ebenso von der vorliegenden Erfindung mit umfasst wie die Auslegung, dass ein Zahlenwort wie beispielsweise "ein" alternativ als "genau ein" gemeint sein kann, wo immer dies für den Fachmann erkennbar technisch möglich ist. Beides ist von der vorliegenden Erfindung umfasst und gilt für alle hierin verwendeten Zahlenworte.

Die Angaben "oben" und "unten" sind hierin bei Zweifel des Fachmanns als absolute oder relative Raumangaben zu verstehen, welche sich auf die Ausrichtung des betreffenden Bauteils während seines üblichen Gebrauchs beziehen.

Die Oberseite der Berührschutzvorrichtung ist im Gebrauch im Zweifel dem Hartteil der Kassette abgewandt, die Unterseite diesem zugewandt.

Erfindungsgemäß ist der erste Verbindungsabschnitt eine Einsteck- oder Rastverbindung oder Teil hiervon.

In bestimmten erfindungsgemäßen Ausführungsformen weist der Abdeckabschnitt wenigstens eine - sich vorzugsweise von der Unterseite der Berührschutzvorrichtung erhebende oder erstreckende - erste Struktur auf, welche in ihrem Umfang geschlossen ist.

In bestimmten erfindungsgemäßen Ausführungsformen weist der Abdeckabschnitt wenigstens eine - sich vorzugsweise von der Unterseite der Berührschutzvorrichtung erhebende oder erstreckende - zweite Struktur auf, welche in ihrem Umfang geschlossen ist.

In bestimmten erfindungsgemäßen Ausführungsformen weist die Berührschutzvorrichtung wenigstens einen Griffabschnitt auf. Der Griffabschnitt weist vorzugsweise wenigstens eine - sich insbesondere von der Unterseite der Berührschutzvorrichtung erhebende oder erstreckende - Struktur auf, welche in ihrem Umfang vorzugsweise geschlossen ist.

In bestimmten erfindungsgemäßen Ausführungsformen weist die die Struktur des Griffabschnitts, die sich optional auch auf den zweiten Verbindungsabschnitt erstrecken kann, wenigstens eine Vertiefung in ihrem äußersten Rand auf.

Eine Vertiefung, wie hierin verwendet, kann ein Abschnitt sein, in welchem die Struktur, welche die Vertiefung aufweist, eine geringere Höhe hat als benachbarte, optional als beide benachbarten, Abschnitte der Struktur. Eine Vertiefung kann eine Einbuchtung, eine Aussparung, ein Einschnitt sein. Eine Vertiefung ist vorzugsweise keine geschlossene Durchgangsöffnung. Eine Vertiefung kann ähnlich einer Schießscharte ausgestaltet sein.

In bestimmten erfindungsgemäßen Ausführungsformen weist die Berührschutzvorrichtung wenigstens einen, dem Griffabschnitt vorzugsweise benachbarten, Abstandshalter auf. Der Abstandshalter steht, vorzugsweise in einer Richtung der Tiefe der Kassette, vorzugsweise von der Unterseite der Berührschutzvorrichtung, von der Berührschutzvorrichtung ab. Der Abstandshalter stellt damit einen Mindestabstand, unterhalb welchem sich manche Abschnitte der Berührschutzvorrichtung, und insbesondere deren Griffabschnitt, einerseits und das Hartteil der Kassette andererseits, nicht näher kommen können, sicher.

Auf diese Weise erlaubt der Abstandshalter dem Benutzer, den Griffabschnitt beidseits, also auf dessen Oberseite wie auch auf dessen Unterseite zwischen den Fingern zu greifen, um die Berührschutzvorrichtung zu entfernen.

In bestimmten erfindungsgemäßen Ausführungsformen steht der Griffabschnitt optional nach vorne (also in einer Richtung der Haupterstreckung der Kassette) über den Rand der Kassette oder deren Hartteils über. Auch dies mag dem Benutzer das Entfernen der Berührschutzvorrichtung erleichtern.

Erfindungsgemäß weist die Berührschutzvorrichtung einen zweiten Verbindungsabschnitt zum lösbaren Verbinden der Berührschutzvorrichtung mit der Kassette oder zum Halten der Berührschutzvorrichtung an der Kassette auf.

Erfindungsgemäß weist der zweite Verbindungsabschnitt eine Erhebung zum Einführen in die Zentrieröffnung der fluidführenden Kassette auf.

In einigen beispielhaften erfindungsgemäßen Ausführungsformen weist die Erhebung außenseitig einen oder mehrere Vorsprünge auf. Diese können eine Rastwirkung beim Verrasten der Erhebung in der Zentrier- oder Haltöffnung begünstigen.

In bestimmten erfindungsgemäßen Ausführungsformen weist der zweite Verbindungsabschnitt eine - sich insbesondere von der Unterseite der Berührschutzvorrichtung erhebende oder erstreckende - Struktur auf, welche in ihrem Umfang vorzugsweise geschlossen ist.

In bestimmten erfindungsgemäßen Ausführungsformen weist die Struktur wenigstens eine Vertiefung in ihrem äußersten Rand auf.

In einigen beispielhaften erfindungsgemäßen Ausführungsformen weist der zweite Verbindungsabschnitt optional wenigstens eine erste Durchgangsöffnung und/oder eine zweite Durchgangsöffnung auf.

In bestimmten erfindungsgemäßen Ausführungsformen ist der Griffabschnitt zwischen dem ersten Verbindungsabschnitt und dem zweiten Verbindungsabschnitt angeordnet und/oder verbindet beide.

In bestimmten erfindungsgemäßen Ausführungsformen weist die Berührschutzvorrichtung im Bereich des zweiten Verbindungsabschnitts wenigstens eine erste Durchgangsöffnung auf. Die Durchgangsöffnungen verbinden beispielsweise die Oberseite der Berührschutzvorrichtung mit deren Unterseite.

In bestimmten erfindungsgemäßen Ausführungsformen weist die Berührschutzvorrichtung im Bereich des ersten Verbindungsabschnitts wenigstens einen Abstandshalter auf.

Der Abstandshalter erstreckt sich beispielsweise benachbart zum Verbindungsabschnitt und in dieselbe Raumrichtung wie letzterer.

Der Abdeckabschnitt wird in manchen erfindungsgemäßen Ausführungsformen mittels - insbesondere ausschließlich - Spannung oder Materialsteifigkeit an der Kassette gehalten. Zwischen dem Abdeckabschnitt und der Kassette besteht in diesen Ausführungsformen keine stoffschlüssige Verbindung.

In bestimmten erfindungsgemäßen Ausführungsformen ist die Berührschutzvorrichtung ausgestaltet und/oder verbunden, um zerstörungsfrei von der Kassette abnehmbar zu sein. Die Berührschutzvorrichtung wird in einigen erfindungsgemäßen Ausführungsformen mittels des ersten Verbindungsabschnitts an der Kassette gehalten. Der Verbindungsabschnitt sorgt für eine lösbare und stabile Verbindung der Berührschutzvorrichtung mit der Kassette. Diese Verbindung kann klebstofffrei erfolgen. Sie kann ein Einrasten, Arretieren, Einbringen, Einklemmen, Einschrauben, ein Halten mittels physikalischer Kräfte oder eine sonstige aus dem Stand der Technik bekannte Art des lösbaren Verbindens sein. Die Berührschutzvorrichtung ist in manchen erfindungsgemäßen Ausführungsformen ausgestaltet, um beliebig oft mit der Kassette verbindbar und wieder lösbar zu sein.

In gewissen Ausführungsformen ist die Berührschutzvorrichtung plattenförmig. In manchen erfindungsgemäßen Ausführungsformen ist die Berührschutzvorrichtung einteilig oder einstückig. Es ist aber für den Fachmann erkennbar, dass die Berührschutzvorrichtung sowohl jede beliebige aus dem Stand der Technik bekannte Form haben kann, um ihre Funktion zu erfüllen, als auch ein- oder mehrstückig oder -teilig gefertigt werden kann.

In manchen erfindungsgemäßen Ausführungsformen wird die Berührschutzvorrichtung allein durch den ersten und ggf. durch einen zweiten Verbindungsabschnitt an der Kassette gehalten.

In manchen erfindungsgemäßen Ausführungsformen weist die Berührschutzvorrichtung wenigstens eine Kodierstruktur zum formbedingt kodierten Anordnen der Berührschutzvorrichtung an der fluidführenden Kassette auf. Das kodierte Anordnen kann zu einer stabilen Verbindung der Berührschutzvorrichtung an der Kassette beitragen. Hierzu hat die Kodierstruktur eine Form, die beispielsweise mit der Form oder Kontur eines Randes (oder eines anderen Abschnitts) der Kassette korreliert oder sich mit dieser ergänzt oder komplementär zu dieser ist. Das bewirkt einerseits, dass die Verbindung zwischen der Berührschutzvorrichtung und der im Randbereich in einer zur Kodierstruktur komplementär verlaufenden Kassette stabil ist. So kann die Kodierstruktur beispielsweise das Verrutschen der Berührschutzvorrichtung oder deren Verdrehen um die Achse ihrer Verbindung mit der Kassette, beispielsweise um den Verbindungsabschnitt, verhindern. Andererseits kann die Kodierstruktur vorteilhaft verhindern, dass die Berührschutzvorrichtung während des Herstellungsprozesses in einer anderen Position zur Kassette als der beabsichtigten an der Kassette angebracht wird. Somit kann ein für den beabsichtigten Berührschutz unwirksames Anbringen der Berührschutzvorrichtung vorteilhaft verhindert werden.

In einigen erfindungsgemäßen Ausführungsformen ist die Kodierstruktur der Berührschutzvorrichtung wellenförmig oder weist einen wellenförmigen Abschnitt auf. Es ist für den Fachmann erkennbar, dass die Kodierstruktur jede erdenkliche Form aufweisen kann, mit welcher sie wenigstens eine der o. g. Funktionen oder Vorteile erfüllen kann. Beispielsweise kann sie eine gerade Linie, ein Zickzackmuster oder sonstiges gestuftes Muster aufweisen. Sie kann ein kontinuierliches oder ein nicht-kontinuierliches Muster aufweisen, usw.

In gewissen erfindungsgemäßen Ausführungsformen ist die Kodierstruktur der Berührschutzvorrichtung eine zu diesem Zweck vorgesehene Aussparung in wenigstens einem Abschnitt oder auf einer Seite der Berührschutzvorrichtung.

In manchen erfindungsgemäßen Ausführungsformen weist die Berührschutzvorrichtung wenigstens eine Fixierungseinrichtung zum lösbaren Aufnehmen, Begrenzen oder Fixieren von Zu- und/oder Ablaufschläuchen der fluidführenden Kassette auf.

Hierzu hält die Fixierungseinrichtung die Schläuche in einigen erfindungsgemäßen Ausführungsformen in einer gewünschten Stellung relativ zur Kassette. Die Fixierungseinrichtung kann so die Beschädigung der Schläuche durch Abknicken oder deren Kontamination durch ungewolltes Lösen der Schläuche von der Kassette verhindern.

In bestimmten erfindungsgemäßen Ausführungsformen ist die Fixierungseinrichtung mehrteilig ausgeführt. Zwischen den einzelnen Abschnitten der mehrteiligen Fixierungseinrichtung können weitere Gegenstände wie beispielsweise Schlauchklemmen, welche wiederum mit den vorgenannten Schläuchen verbunden sein können, vorübergehend mittels Reibhaftung oder Rastwirkung gehalten werden. Dies kann einem Schutz der Gegenstände wenigstens bis zum Gebrauch der Kassette dienen.

In einigen erfindungsgemäßen Ausführungsformen ist die Fixierungseinrichtung auf nur einer Seite der Berührschutzvorrichtung angeordnet. Dabei erstreckt sie sich nur auf eine Seite.

In manchen erfindungsgemäßen Ausführungsformen weist die Berührschutzvorrichtung eine, im Wesentlichen ebene, vollständig ebene oder ausschließlich ebene Fläche auf, in anderen Ausführungsformen jedoch nicht. Diese Fläche ist im Gebrauch der Berührschutzvorrichtung der Konnektionsstelle abgewandt. Die ebene Fläche verhindert vor dem Gebrauch der Kassette vorteilhaft ein ungewolltes Verhaken der Berührschutzvorrichtung mit anderen Gegenständen oder ihr Hängenbleiben an diesen, wodurch die Berührschutzvorrichtung unbeabsichtigt von der Kassette gelöst werden könnte.

In gewissen erfindungsgemäßen Ausführungsformen weist die Berührschutzvorrichtung wenigstens einen Griffabschnitt zum Greifen der Schutzvorrichtung auf, um die Berührschutzvorrichtung mit der Hand von der fluidführenden Kassette zu lösen, in anderen Ausführungsformen jedoch nicht. Mittels des Griffabschnitts entfernt oder löst der Benutzer die Berührschutzvorrichtung von der Kassette, z. B. vor dem Einlegen der Kassette in eine Fluidbehandlungsvorrichtung.

Der Griffabschnitt kann jede dem Stand der Technik bekannte Ausgestaltung aufweisen, insbesondere eine solche, welche ein rutschsicheres Halten oder Greifen des Griffabschnittes erleichtert oder ermöglicht. Beispielsweise kann der Griffabschnitt als Ring, Noppen, Lasche usw. ausgestaltet sein oder eine Riffelung oder raue Oberfläche aufweisen.

In manchen erfindungsgemäßen Ausführungsformen liegt der Abdeckabschnitt nicht auf dem Griffabschnitt vor, sondern auf einem anderen Abschnitt der Berührschutzvorrichtung. Das bewirkt, dass der Abdeckabschnitt nicht angefasst werden muss, um die Berührschutzvorrichtung von der Kassette zu lösen. Dies minimiert somit die Gefahr der versehentlichen Kontamination während des Lösens der Berührschutzvorrichtung.

In bestimmten Ausführungsformen der Berührschutzvorrichtung liegt der Griffabschnitt auf einem dem Abdeckabschnitt entgegengesetzten Abschnitt der Berührschutzvorrichtung vor. Dies kann zu einem kontaminationslosen Lösen der Berührschutzvorrichtung von der Kassette beitragen.

In manchen erfindungsgemäßen Ausführungsformen der medizinischen fluidführenden Kassette ist die Berührschutzvorrichtung klebstofffrei mit der Kassette verbunden.

In einigen erfindungsgemäßen Ausführungsformen weist die Berührschutzvorrichtung einen Abschnitt auf, welcher über einen Rand der Kassette hinausragt.

In manchen erfindungsgemäßen Ausführungsformen ist der Griffabschnitt der Berührschutzvorrichtung auf dem über den Rand der Kassette hinausragenden Abschnitt angeordnet, um dem Benutzer das Entfernen der Berührschutzvorrichtung von der Kassette zu erleichtern.

Erfindungsgemäß kann der zweite Verbindungsabschnitt in eine Zentrieröffnung der Kassette eingeführt werden, um die Berührschutzvorrichtung - ggf. hierdurch unterstützt - mit der Kassette zu verbinden. Diese Zentrieröffnung der Kassette kann vorgesehen sein, um mit einer Zentriereinrichtung einer Fluidbehandlungsvorrichtung verbunden zu werden. Sie dient beispielsweise dem Zentrieren der Kassette an der Fluidbehandlungsvorrichtung im Gebrauch, beispielsweise während einer Dialysebehandlung.

In manchen erfindungsgemäßen Ausführungsformen verhindert der Verbindungsabschnitt der Berührschutzvorrichtung im Gebrauch das Anbringen der Kassette an einer Fluidbehandlungsvorrichtung oder das Einlegen der Kassette hierin. Dies wird beispielsweise dadurch erreicht, dass, wenn die Berührschutzvorrichtung mit der Kassette verbunden ist, der Verbindungsabschnitt in der Zentrieröffnung der Kassette steckt. Da die Kassette mit aufgesetzter Berührschutzvorrichtung nicht an der Fluidbehandlungsvorrichtung angebracht werden kann, weil ihre Zentrieröffnung nicht frei liegt, muss die Berührschutzvorrichtung vorher bewusst entfernt werden.

In weiteren erfindungsgemäßen Ausführungsformen ist die Berührschutzvorrichtung derart ausgestaltet, beispielsweise durch ihre Dimensionen oder ihre Art der Verbindung mit der Kassette, dass die Kassette mit aufgesetzter Berührschutzvorrichtung zwar an der Fluidbehandlungsvorrichtung angebracht werden kann, jedoch etwa eine Abdeckung, oder beispielsweise eine Maschinentür der Vorrichtung, nicht geschlossen werden kann, ohne dass zuvor die Berührschutzvorrichtung von der Kassette gelöst wurde. Auch dies dient dazu sicherzustellen, dass die Berührschutzvorrichtung vor Gebrauch der Kassette zuverlässig entfernt wird.

In einigen vorteilhaften erfindungsgemäßen Ausführungsformen ist die Berührschutzvorrichtung zusammen mit der Kassette sterilisiert. Das Sterilisationsverfahren der Kassette findet in diesen Ausführungsformen mit aufgesetzter Berührschutzvorrichtung statt. Dies ist erfindungsgemäß möglich, da in solchen Ausführungsformen zwischen dem Abdeckabschnitt der Berührschutzvorrichtung - in ihrem über der Konnektionsstelle angeordneten Zustand - und der Kassette und/oder der Konnektionsstelle keine stoffschlüssige oder dampfundurchlässige Verbindung besteht. Somit wird z. B. die Dampfdurchgängigkeit der Berührschutzvorrichtung sichergestellt. Die Konnektionsstelle kann selbst bei aufgesetzter/m Berührschutzvorrichtung bzw. Abdeckabschnitt erfolgreich sterilisiert werden, beispielsweise mit Heißdampf.

Dieser Vorteil kann sich auch ergeben, wenn Berührschutzvorrichtung und Kassette aus demselben Material oder aus ähnlichen Materialien sind, jedenfalls aus Materialien, welche mit demselben Sterilisationsverfahren, mit welchem auch die Kassette sterilisierbar ist, sterilisiert werden können.

In manchen erfindungsgemäßen Ausführungsformen ist die wenigstens eine Konnektionsstelle, welche mittels des Abdeckabschnitts abgedeckt ist oder wird, eine Verbindungsstelle für eine Substituatleitung.

In einer weiteren erfindungsgemäßen Ausführungsform ist die medizinische fluidführende Kassette als Blutkassette, beispielsweise für die Dialysebehandlung, insbesondere zur Hämodialyse, zur Hämofiltration, zur Hämodiafiltration, zur Peritonealdialyse, zur Akutdialyse, usw. ausgestaltet.

In einigen vorteilhaften erfindungsgemäßen Ausführungsformen ist die Tiefe der Zentrieröffnung der fluidführenden Kassette größer als die Höhe der Erhebung des zweiten Verbindungsabschnitts.

In einigen vorteilhaften erfindungsgemäßen Ausführungsformen weist die Kassette Erhebungen im Bereich des zweiten Verbindungsabschnitts auf, deren Geometrien der Form der ersten und/oder der zweiten Durchgangsöffnung angepasst sind.

In einigen erfindungsgemäßen Ausführungsformen bildet die erste Struktur der Berührschutzvorrichtung oder des Abdeckabschnitts, die wie ein konzentrischer Ring um das Zentrum des Abdeckabschnitts angeordnet ist, zusammen mit einer rohrförmigen Kanalstruktur der Konnektionsstelle der Kassette eine Labyrinthdichtung, indem zwei ringförmige Strukturen ineinander gesteckt sind.

In einigen, beispielhaften erfindungsgemäßen Ausführungsformen sind unter dem Hartteil (auch als Hartkörper oder als Substrat bezeichnet) der in der Regel in einem Spritzgussverfahren hergestellte und daher hierin als "hart" bezeichnete Korpus der medizinischen Einrichtung, welcher mit einer vergleichsweise "weichen" Folie bedeckt sein kann, und/oder seine Anhänge wie Schläuche usw., zu verstehen. Das Hartteil kann aus PP (Polypropylen), PE (Polyethylen), PA, ABS, PMMA, PC, PVC oder anderen dem Fachmann hinlänglich bekannten Polymeren oder anderen Materialien gefertigt sein. Es kann aus Isolatormaterialien, wie insbesondere z. B. Keramik, gefertigt sein.

In einigen beispielhaften erfindungsgemäßen Ausführungsformen weist die erste Struktur des Abdeckabschnitts, die zweite Struktur des Abdeckabschnitts, die umlaufende Struktur des zweiten Verbindungsabschnitts und/oder die umlaufende Struktur des Griffabschnitts eine oder mehrere Vertiefungen, Einschnitte oder Absenkungen auf.

In einigen beispielhaften erfindungsgemäßen Ausführungsformen ist die Fixiereinrichtung als Halter für den Rinse-Port-Adapter ausgestaltet, wobei vorgesehen ist, dass letzterer in ersten eingesteckt wird.

In einigen beispielhaften erfindungsgemäßen Ausführungsformen weist die Fixiereinrichtung ein, zwei oder mehr Gelenke, insbesondere Filmgelenke auf, mittels welcher die Fixiereinrichtung mit dem Hauptkörper der Berührschutzvorrichtung verbunden ist, z. B. mit dem zweiten Verbindungsabschnitt.

In einigen beispielhaften erfindungsgemäßen Ausführungsformen weist die Fixierungseinrichtung einen zylinderförmigen Einsteckabschnitt auf, der von einer ringförmigen Struktur umgeben ist. Zwischen dem zylinderförmigen Einsteckabschnitt und der ringförmigen Struktur liegt ein Spalt, in welchem das vordere Ende des Rinse-Port-Adapters, nach oder durch Einstecken und ggf. Verklemmen darin, gehalten werden kann.

In einigen beispielhaften erfindungsgemäßen Ausführungsformen weist die Fixierungseinrichtung optional drei kreisförmige Strukturen oder Flächen auf.

In einigen vorteilhaften Ausführungsformen weist die Berührschutzvorrichtung eine Drainagestruktur auf.

In einigen vorteilhaften Ausführungsformen weist die Drainagestruktur Rillen oder Nuten auf, oder besteht hieraus, welche sich z. B. radial von der Fixierungseinrichtung, hier z. B. dem Einsteckabschnitt zur Aufnahme z. B. eines Rinse-Port-Adapters, erstrecken.

In einigen vorteilhaften Ausführungsformen erstrecken sich die Rillen oder Nuten bodenseitig zwischen dem erhabenen Einsteckabschnitt und der ringförmigen Struktur, die den Einsteckabschnitt umgibt.

Manche oder alle erfindungsgemäßen Ausführungsformen können einen, mehrere oder alle der oben und/oder im Folgenden genannten Vorteile aufweisen.

Vorteilhaft ist das Lösen der Berührschutzvorrichtung ohne nennenswerte Kraftanwendung aufgrund der o. g. Ausgestaltung der Berührschutzvorrichtung möglich.

In einigen vorteilhaften Ausführungsformen besteht ein vermindertes Risiko der unbeabsichtigten Kontamination der Konnektionsstelle der Kassette, etwa durch Berühren mit der Hand, da der Griffabschnitt der Berührschutzvorrichtung ausreichend weit vom Abdeckabschnitt beabstandet ist.

Die Berührschutzvorrichtung ist in vorteilhaften Ausführungsformen aus demselben Material wie die Kassette gefertigt. Somit kann die Berührschutzvorrichtung denselben Sterilisationsverfahren wie die Kassette unterzogen werden. Zudem kann sie auf die gleiche Weise wie die Kassette oder gemeinsam mit dieser entsorgt werden.

Das Lösen der Berührschutzvorrichtung von der Kassette kann in vorteilhaften Ausführungsformen einhändig erfolgen.

In bestimmten erfindungsgemäßen Ausführungsformen weist die Berührschutzvorrichtung entsprechend weiche oder flexible Materialien oder gerundete Kanten derart auf, dass eine Verletzungsgefahr für den Benutzer während ihrer Betätigung, sowie die Gefahr der Beschädigung anderer Bestandteile der Berührschutzvorrichtung oder der Kassette durch beispielsweise scharfe Konturen minimiert sind.

Durch den möglichen Verzicht auf die Verwendung von Klebstoff zum Anbringen der Berührschutzvorrichtung an der Kassette können bei ihrem Lösen von der Kassette keine Klebstoffreste an der Kassette verbleiben und ggf. über die Konnektionsstelle mit in das Kassetteninnere gelangen.

Anders als z. B. bei der Verwendung von Sterilitätskappen besteht erfindungsgemäß keine Gefahr des versehentlichen Berührens der Konnektionsstelle beim Auspacken der Kassette.

Anders als bei Schutzfolien und Sterilitätskappen kann die erfindungsgemäße Berührschutzvorrichtung beim Sterilisieren der Kassette aufgesetzt sein. Ein separates Sterilisieren von Berührschutzvorrichtung und Kassette mit anschließendem Zusammenfügen ist vorteilhaft nicht erforderlich.

Die vorliegende Erfindung wird im Folgenden anhand der beigefügten Zeichnung, in welcher gleiche Bezugszeichen gleiche oder ähnliche Bauteile bezeichnen, exemplarisch erläutert. In den Figuren gilt:
- Fig. 1: zeigt die Berührschutzvorrichtung in einer ersten Ausführungsform von schräg unten, mit Blick auf ihre im Gebrauch zur fluidführenden Kassette hin gewandte Unterseite;
- Fig. 2: zeigt die Berührschutzvorrichtung der Fig. 1 von schräg oben (also mit Blick auf die Oberseite), verbunden mit der nur abschnittsweise dargestellten Kassette;
- Fig. 3: zeigt die Berührschutzvorrichtung der Fig. 1 und 2 von unten, verbunden mit der nur abschnittsweise dargestellten Kassette;
- Fig. 4: zeigt in einer der Fig. 2 ähnlichen perspektivischen Darstellung die Berührschutzvorrichtung 100 in einem Längsschnitt, verbunden mit der nur abschnittsweise dargestellten Kassette;
- Fig. 5: zeigt eine perspektivische Darstellung der Berührschutzvorrichtung der vorangegangenen Figuren im Gebrauch an einer fluidführenden Kassette;
- Fig. 6a-6c: zeigen den ersten Verbindungsabschnitt der Berührschutzvorrichtung der ersten Ausführungsform in drei Stadien des Übergangs vom verrasteten Zustand (Fig. 6a) bis zum entrasteten Zustand (Fig. 6c);
- Fig. 7: zeigt die erfindungsgemäße Berührschutzvorrichtung in einer zweiten Ausführungsform;
- Fig. 8: zeigt die Berührschutzvorrichtung der Fig. 7, mit auf die Fixierungseinrichtung aufgestecktem Rinse-Port-Adapter;
- Fig. 9: zeigt die Berührschutzvorrichtung in Perspektive und von der Unterseite in einer dritten Ausführungsform mit einer Halte- oder Fixierungseinrichtung;
- Fig. 10: zeigt die Berührschutzvorrichtung der Fig. 9 in einem Schnitt, mit leicht perspektivischem Blick auf die Schnittebene;
- Fig. 11: zeigt die Berührschutzvorrichtung der Fig. 9 und 10, verbunden mit dem Hartteil der Kassette; und
- Fig. 12: zeigt einen Teil der Berührschutzvorrichtung in Perspektive und von der Unterseite in einer vierten Ausführungsform.

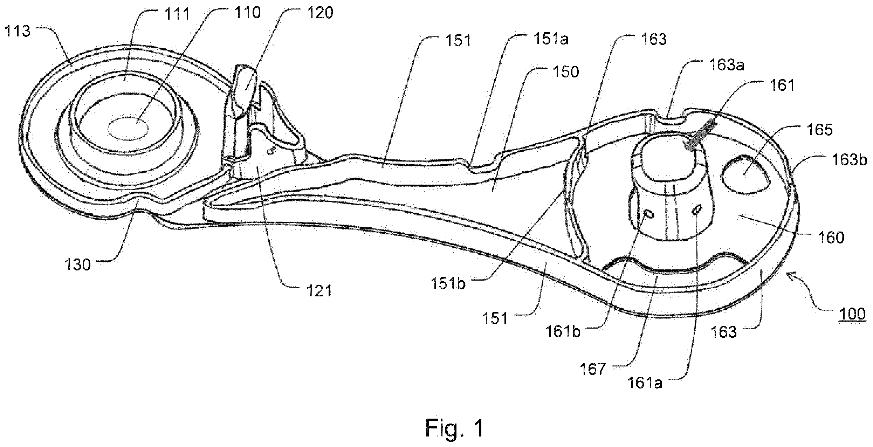

**Fig. 1** zeigt eine mögliche, erste Ausgestaltung der Berührschutzvorrichtung 100. Die Berührschutzvorrichtung 100 weist einen Abdeckabschnitt 110, einen Verbindungsabschnitt 120, eine optionale Kodierstruktur 130 und einen Greif- oder Griffabschnitt 150 auf.

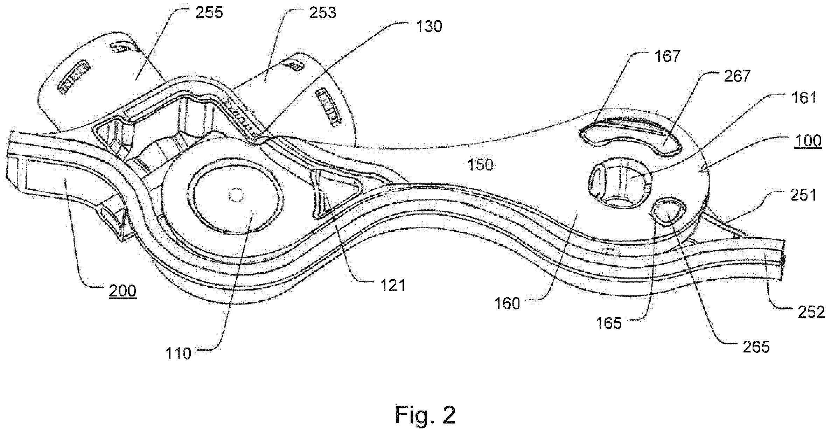

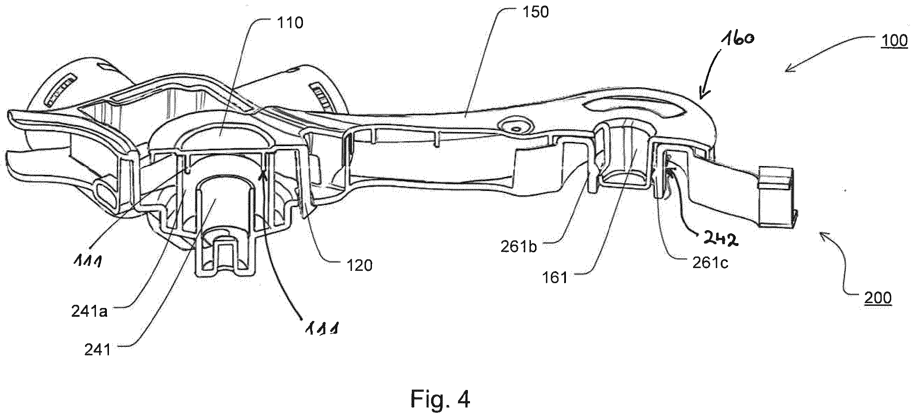

Dabei dient der Abdeckabschnitt 110 im Gebrauch dazu, eine in Fig. 1 nicht gezeigte Konnektionsstelle 241 (siehe Fig. 4) abzudecken. Der Abdeckabschnitt 110 ist breit genug, um die Konnektionsstelle 241 einer fluidführenden Kassette 200 (in Fig. 1 ebenfalls nicht dargestellt, siehe aber Fig. 2 bis Fig. 6c), mittels welcher eine Fluidverbindung zur Kassette 200 hergestellt werden kann, komplett abzudecken. Mittels des Abdeckabschnitts 110 wird somit ein unbeabsichtigtes Berühren der Konnektionsstelle 241 der Kassette 200 vor deren Gebrauch, d. h. vor dem Anbringen der Kassette 200 an einer Fluidbehandlungsvorrichtung, verhindert. Mittels des Abdeckabschnitts 110 wird ein unbeabsichtigtes Berühren der Konnektionsstelle 241 vorzugsweise auch noch während des Entfernens der Berührschutzvorrichtung 100 verhindert.

Der Abdeckabschnitt 110 wird mittels Spannung oder über die Materialsteifigkeit der Berührschutzvorrichtung 100 an der Kassette 200 gehalten. Die Berührschutzvorrichtung 100 wird wiederum mittels - z. B. und/oder u. a. - des Verbindungsabschnitts 120 an einem Hartteil der Kassette 200 gehalten.

Der Verbindungsabschnitt 120 sorgt für eine lösbare und stabile Verbindung der Berührschutzvorrichtung 100 an der Kassette 200. Diese Verbindung ist klebstofffrei und kann ein Einrasten, Arretieren, Einbringen, Einklemmen, Einschrauben oder eine sonstige aus dem Stand der Technik bekannte Art des lösbaren Verbindens sein. Der Verbindungsabschnitt 120 ist im Beispiel der Fig. 1 eine Einsteck- oder Rastverbindung.

In einigen erfindungsgemäßen Ausführungsformen wird die Berührschutzvorrichtung 100 allein durch den Verbindungsabschnitt 120 an der Kassette 200 gehalten. In anderen, wie dies auch in den Figuren gezeigt ist, ist ein zweiter Verbindungsabschnitt 160 vorgesehen.

Zwischen dem Abdeckabschnitt 110 und der Kassette 200 besteht in manchen erfindungsgemäßen Ausführungsformen keine stoffschlüssige Verbindung.

Die Berührschutzvorrichtung 100 weist um den Abdeckabschnitt 110 herum eine erste Struktur 111 auf. Letztere ist als geschlossene Struktur, d. h. mit durchgehend umlaufendem, also nicht-unterbrochenem, Umfang ausgestaltet.

Eine optionale, zweite Struktur 113 umgibt die erste Struktur 111 und ist, wiederum optional, ebenfalls als geschlossene Struktur ausgestaltet.

Während die erste Struktur 111 vorzugsweise einer Abdichtung dient, was den Fig. 4 bis 6d zu entnehmen ist, dient die zweite Struktur 113 vorzugsweise der Versteifung der Berührschutzvorrichtung 100 und insbesondere des Abdeckabschnitts 110.

Ferner kann die zweite Struktur 113 einem Zentrieren oder Ausrichten der Berührschutzvorrichtung 100 auf der Kassette 200 dienen, was wiederum dazu beitragen kann, sicherzustellen, dass die Berührschutzvorrichtung 100 korrekt auf die Kassette 200 aufgesetzt ist.

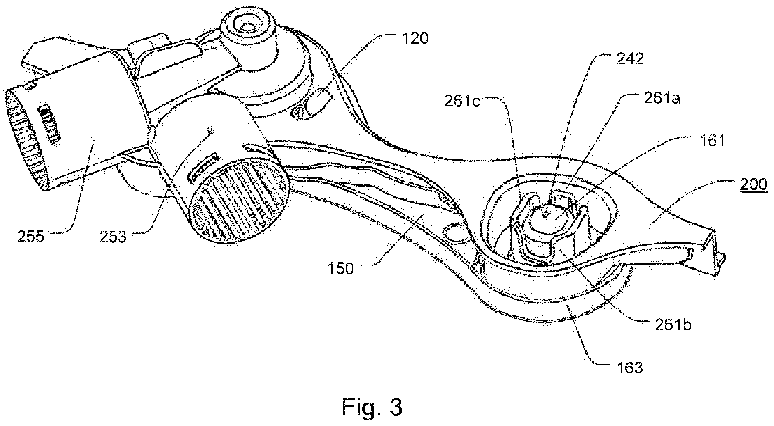

Der erste Verbindungsabschnitt 120 ist hier exemplarisch als Rastabschnitt ausgestaltet, der in eine entsprechende Öffnung oder Durchgangsöffnung (in Fig. 3 gezeigt) der Kassette 200 eingreifen und darin verrasten kann.

Weiter zeigt das Beispiel der Fig. 1 einen optional vorgesehenen Abstandshalter 121. Er stellt optional einen gewünschten Abstand zwischen Berührschutzvorrichtung 100 und Kassette 200 sicher.

Die Form des Abstandshalters 121 ist hier optional im Querschnitt winkelförmig oder dreieckig. Diese Form dient wiederum dem Zentrieren oder Ausrichten der Berührschutzvorrichtung 100 auf der Kassette 200; sie ist an eine geometrische Besonderheit des Hartteils der Kassette 200 angepasst bzw. entspricht dieser. Der Abstandshalter 121 kann in dieser konkreten Ausführungsform daher ebenfalls als Kodierstruktur verstanden werden.

Der Griffabschnitt 150 weist eine Struktur 151 auf, die hier optional ähnlich der o. g. ersten und zweiten Strukturen 111 oder 113 mit geschlossenem Umfang ausgestaltet sein kann.

Der Umfang der Struktur 151 kann ferner optional ausnahmslos erhaben sein, also über einer Bodenfläche stehen. Dies ergibt eine erhöhte Steifigkeit entlang des gesamten Griffabschnitts 150 oder gar der gesamten Berührschutzvorrichtung 100, was ein Abnehmen der Berührschutzvorrichtung 100 von der Kassette 200 ohne Verkanten oder Verklemmen sicherstellen kann, gleich, an welcher Stelle der Benutzer zum Abnehmen der Berührschutzvorrichtung 100 von der Kassette 200 mit seinen Fingern auf den Griffabschnitt 150 einwirkt oder diesen anfasst.

Die Struktur 151 kann eine oder mehrere Vertiefungen, ähnlich oder gleich der hier exemplarisch gezeigten Vertiefungen 151a und/oder 151b, aufweisen. Solche Vertiefungen, an welchen der aufstehende Rand der Struktur 151 niedriger ist als an den Vertiefungen der benachbarten Abschnitte der Struktur 151, kann wiederum dem Zentrieren oder Ausrichten der Berührschutzvorrichtung 100 auf der Kassette 200 über Stegen des Hartteils der Kassette 200 dienen, siehe z. B. den Steg 251 der Fig. 2.

Zudem können Vertiefungen wie die hier exemplarisch gezeigten Vertiefungen 151a und/oder 151b ein Anordnen der Berührschutzvorrichtung 100 auf einem Niveau des oberen Abschlusses des Hartteils, etwa im Bereich seiner umlaufenden Struktur 252, an welcher eine in den Figuren nicht gezeigte Folie mit dem Hartteil verschweißt ist, erlauben. Dies erlaubt es, die Dicke der Kassette 200, auf welche die Berührschutzvorrichtung 100 aufgesetzt ist, vorteilhaft gering zu halten.

Die Berührschutzvorrichtung 100 weist in der exemplarischen Ausführungsform der Fig. 1 ferner wenigstens den vorstehend erwähnten zweiten Verbindungsabschnitt 160 auf. Der zweite Verbindungsabschnitt 160 kann - wie der erste Verbindungsabschnitt 120 - die Berührschutzvorrichtung 100 lösbar mit der Kassette 200 verbinden.

Der zweite Verbindungsabschnitt 160 weist hierzu eine Erhebung 161 auf. Sie kann, wie im Beispiel der Fig. 1, entsprechend geformt sein, um in eine Zentrieröffnung 242 der Kassette 200 eingesteckt zu werden, die in Fig. 3 gezeigt ist. Dabei kann die Zentrieröffnung 242 mit einer beliebigen Anzahl an Rastnasen oder Nasen 261a, 261b, 261c ausgestaltet sein, die der lösbaren Verbindung zwischen Erhebung 161 und Zentrieröffnung 242 dienen können.

Die Erhebung 161 kann außenseitig Vorsprünge wie die hier exemplarisch gezeigten Vorsprünge 161a, 161b aufweisen, die dem Verrasten dienen können.

Der zweite Verbindungsabschnitt 160 kann eine Struktur 163 aufweisen, die hier optional ähnlich der o. g. ersten und zweiten Strukturen 111 oder 113 mit geschlossenem Umfang ausgestaltet sein kann.

Auch die Struktur 163 kann eine oder mehrere Vertiefungen 163a, 163b mit o. g. Vorteilen und Ausgestaltungen aufweisen.

Fig. 1 zeigt schließlich, dass der zweite Verbindungsabschnitt 160 optional wenigstens eine erste Durchgangsöffnung 165 und eine zweite Durchgangsöffnung 167 aufweist. Diese können der Aufnahme von Erhebungen 265, 267 (siehe Fig. 2) der Kassette 200 dienen. Sie können damit dem Zentrieren und Ausrichten wie vorstehend beschrieben dienen.

Die Kodierstruktur 130 trägt zu einer stabilen Verbindung der Berührschutzvorrichtung 100 an der Kassette 200 bei.

Die Kodierstruktur 130 hat eine Form, die mit der Form oder Kontur eines Randes oder eines Abschnitts des Hartteils der Kassette 200 korreliert oder sich mit diesem ergänzt oder komplementär zu diesem ist.

Alle vorgenannten Strukturen sind beim Beispiel der Fig. 1 ausschließlich auf der Unterseite der Berührschutzvorrichtung 100 vorgesehen.

**Fig. 2** zeigt die Berührschutzvorrichtung 100 der Fig. 1 von schräg oben (also mit Blick auf die Oberseite).

In Fig. 2 ist die Berührschutzvorrichtung 100 bestimmungsgemäß auf der Kassette 200, welche nur abschnittsweise gezeigt ist, lösbar angebracht. Dabei deckt der Abdeckabschnitt 110 die Konnektionsstelle 241 schützend ab.

Zu erkennen sind die Konnektoren 253 und 255 zur Verbindung des optionalen Substituatschlauchs mit der Kassette 200.

Wie Fig. 2 zu entnehmen ist, weist die Berührschutzvorrichtung 100 in einigen erfindungsgemäßen Ausführungsformen auf einer Seite (hier auf der Oberseite) überhaupt keine erhabenen Strukturen auf, jedenfalls keine sich über eine Haupterstreckungsebene der Oberseite erhebenden Strukturen.

**Fig. 3** zeigt die Berührschutzvorrichtung 100 der Fig. 1 und 2 von unten, verbunden mit der nur abschnittsweise dargestellten Kassette 200.

Der erste Verbindungsabschnitt 120 ragt mit einem Endabschnitt durch eine Öffnung im Hartteil der Kassette 200, wobei er im Beispiel der Fig. 3 mit dem Hartteil verrastet.

Unter Anfassen des Griffabschnitts 150 entfernt oder löst der Benutzer die Berührschutzvorrichtung 100 vor dem Einlegen der Kassette 200 in eine Fluidbehandlungsvorrichtung von der Kassette 200.

Zu erkennen ist, dass die Erhebung 161 optional von (hier optional drei) elastischen Nasen 261a, 261b und 261c lösbar in der Zentrieröffnung 242 gehalten wird. Die Zentrieröffnung 242 ist hier optional von den Rastnasen oder Nasen 261a, 261b, 261c umgeben oder begrenzt.

**Fig. 4** zeigt in einer perspektivischen Darstellung, welche jener der Fig. 2 ähnlich ist, die Berührschutzvorrichtung 100 in einem Längsschnitt.

Der erste Verbindungsabschnitt 120 ist in Fig. 4 ebenso wie der zweite Verbindungsabschnitt 160 (siehe dessen Erhebung 161) mit dem Hartteil der Kassette 200 verrastet. Die Erhebung 161 wird in einem unteren Bereich von den elastischen Nasen 261a (nicht sichtbar), 261b und 261c lösbar gehalten. Diese haben optional in einem Querschnitt zulaufende Enden als Rastvorsprünge (siehe Fig. 4), welche die Längsbewegung der in Fig. 1 gezeigten Vorsprünge oder Rastvorsprünge 161a, 161b hemmen.

Fig. 4 entnimmt man ferner, dass die Tiefe der Zentrieröffnung 242, zu welcher hier auch die elastischen Nasen 261a, 261b und 261c beitragen können, größer ist als die Höhe der Erhebung 161 des zweiten Verbindungsabschnitts 160. Daher steht die Erhebung 161 nicht (bezogen auf die Anordnung in Fig. 4) nach unten aus der Zentrieröffnung 242 hervor. Ein mit diesem Größenverhältnis zwischen der Tiefe der Zentrieröffnung 242 und der Höhe der Erhebung 161 erzielbarer Vorteil kann darin bestehen, dass die Berührschutzvorrichtung 100 nicht ungewollt durch versehentlichen Druck auf die aus der bei anderer Ausgestaltung aus der Zentrieröffnung 242 hervorstehende Erhebung 161 entfernt werden kann.

Zu erkennen ist weiter, dass die erste Struktur 111, die wie ein konzentrischer Ring um das Zentrum des Abdeckabschnitts 110 angeordnet ist, zusammen mit einer rohrförmigen Kanalstruktur 241a der Konnektionsstelle 241 der Kassette 200 eine (optional simple) Labyrinthdichtung bildet, indem zwei ringförmige Strukturen ineinander gesteckt sind.

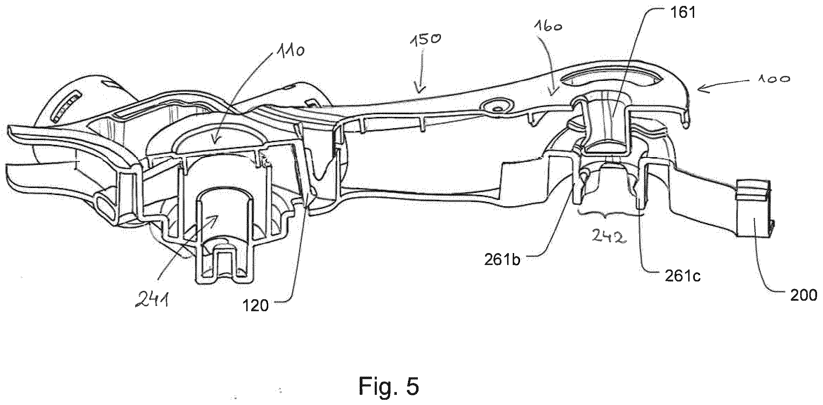

**Fig. 5** zeigt die Berührschutzvorrichtung 100 in der Darstellung der Fig. 4, gerade nicht mehr verbunden mit dem Hartteil der Kassette 200. Die Verrastung mit dem Hartteil, die in Fig. 4 noch gegeben ist, ist in Fig. 5 erkennbar gelöst. Dies betrifft sowohl den ersten Verbindungsabschnitt 120 als auch den optionalen, zweiten Verbindungsabschnitt 160. So steht die Labyrinthdichtung des ersten Verbindungsabschnitts 120 kurz davor, sich aufzulösen. Der bodenseitige Abschnitt der Erhebung 161 ist bereits an den Rastvorsprüngen der elastischen Nasen 261a (nicht sichtbar), 261b und 261c vorbei nach oben geführt.

Wenn die Berührschutzvorrichtung 100 mit der Kassette 200 verbunden ist, wie dies in Fig. 4 gezeigt ist, steckt der zweite Verbindungsabschnitt 160 in der Zentrieröffnung 242 oder Halteöffnung der Kassette 200, vorgesehen zu ihrem Zentrieren oder Halten in einer Fluidbehandlungsvorrichtung. Die Kassette 200 kann in diesem Zustand nicht an der Fluidbehandlungsvorrichtung angebracht werden, da die Zentrieröffnung 242 nicht frei zugänglich liegt. Da allerdings ein freier Zugang zur Zentrieröffnung 242 Voraussetzung für das Anbringen der Kassette 200 an der Fluidbehandlungsvorrichtung ist, muss die Berührschutzvorrichtung 100 bewusst entfernt werden.

Die Berührschutzvorrichtung 100 kann auch derart ausgestaltet sein, beispielsweise durch ihre Dimensionen oder ihre Art der Verbindung zur Kassette 200, dass die Kassette 200 mit aufgesetzter Berührschutzvorrichtung 100 zwar an der Fluidbehandlungsvorrichtung angebracht werden kann, jedoch etwa die Tür der Vorrichtung nicht geschlossen werden kann, bevor die Berührschutzvorrichtung 100 von der Kassette 200 gelöst wird. Auch dies dient dazu, sicherzustellen, dass die Berührschutzvorrichtung 100 vor Gebrauch der Kassette 200 zuverlässig entfernt wurde.

Das Lösen der Berührschutzvorrichtung 100 erfolgt ohne nennenswerte Kraftanwendung. Es erfolgt ferner unter vermindertem Risiko der unbeabsichtigten Kontamination der Konnektionsstelle 241 der Kassette 200, etwa durch Berühren mit der Hand, da der Griffabschnitt 150 der Berührschutzvorrichtung 100 vom Abdeckabschnitt 110 beabstandet ist.

Der Griffabschnitt 150 steht nicht zuletzt wegen des Abstandshalters 121 in einer Richtung der Tiefe der Kassette 200 (also in der Richtung, in welcher sich die Erhebung 161 längs erstreckt) vom Hartteil der Kassette 200 ab oder steht unter Abstand zum Hartteil. Dies erlaubt es dem Benutzer, den Griffabschnitt 150 beidseits, also sowohl auf dessen Oberseite wie als auch auf dessen Unterseite zwischen den Fingern zu greifen, um die Berührschutzvorrichtung 100 zu entfernen.

Ferner steht der Griffabschnitt 150 über den Rand der Kassette 200 optional nach vorne (also in einer Richtung der Haupterstreckung der Kassette 200) über. Auch dies dient dem Benutzer, das Entfernen der Berührschutzvorrichtung 100 zu erleichtern.

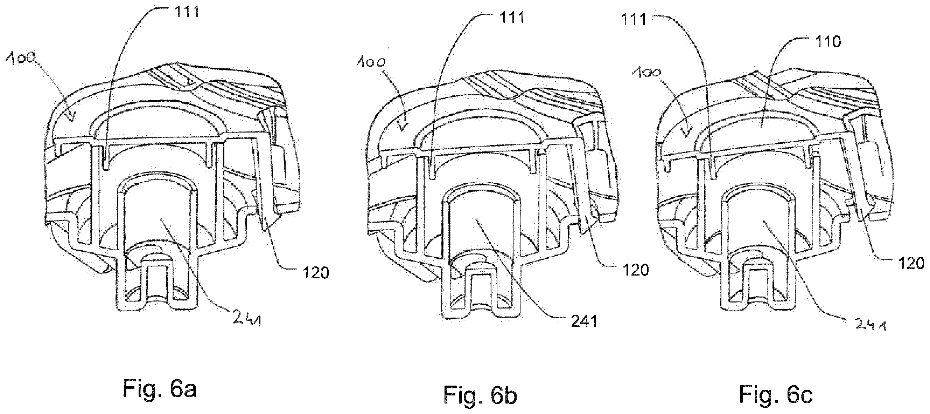

**Fig. 6a bis 6c** zeigen den ersten Verbindungsabschnitt 120 der Berührschutzvorrichtung 100 der ersten Ausführungsform in drei Stadien des Übergangs vom verrasteten Zustand (Fig. 6a) bis zum entrasteten Zustand (Fig. 6c).

Gut zu erkennen ist anhand der Abfolge der Fig. 6a bis 6c, dass der als Rastarm ausgestaltete Verbindungsabschnitt 120 im Verbindungszustand unter Biegevorspannung verrastet ist. Dies kann es vorteilhaft erlauben, eine Abstandstoleranz zwischen der Konnektionsstelle 241 und der Zentrieröffnung 242 oder Halteöffnung zu kompensieren.

In Fig. 6c ist ferner zu erkennen, dass die Labyrinthdichtung, die aus oder mit der ersten Struktur 111 und der rohrförmigen Kanalstruktur 241a ausgestaltet ist, mit ausreichendem radialem Abstand zwischen der ersten Struktur 111 und der rohrförmigen Kanalstruktur 241a versehen oder vorgesehen ist, um ebenfalls eine Toleranz im Abstand zwischen der Konnektionsstelle 241 und der Zentrieröffnung 242 oder Halteöffnung zu kompensieren.

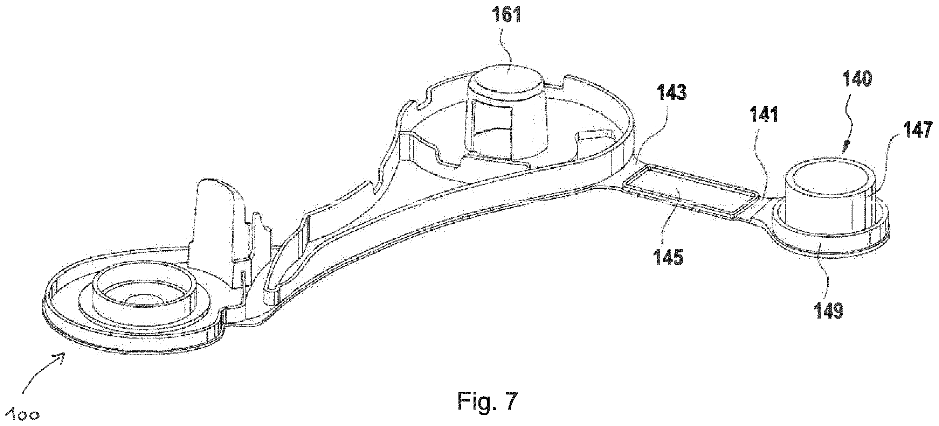

**Fig. 7** zeigt die Berührschutzvorrichtung 100 in einer zweiten Ausführungsform mit einer Halte- oder Fixierungseinrichtung 140. Abgesehen von der Fixierungseinrichtung 140 unterscheidet sich die Berührschutzvorrichtung 100 der Fig. 7 nicht von jener der vorangegangenen Figuren.

Die Fixierungseinrichtung 140 ist optional vorgesehen. Sie dient, falls vorhanden, zum direkten oder indirekten, lösbaren Aufnehmen beispielsweise von Zu- und Ablaufschläuchen. Die Fixierungseinrichtung 140 hält solche Schläuche in einer gewünschten Stellung zur Kassette 200. Sie kann so die Beschädigung der Schläuche durch Abknicken verhindern, ebenso deren Kontamination durch ungewolltes Lösen der Schläuche von der Kassette 200.

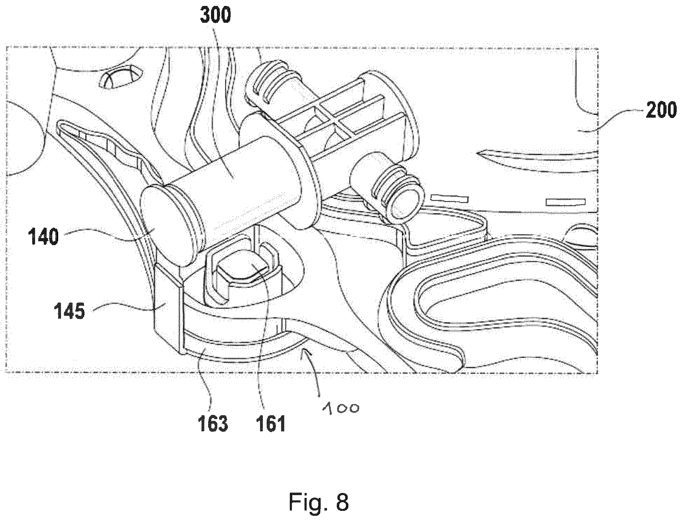

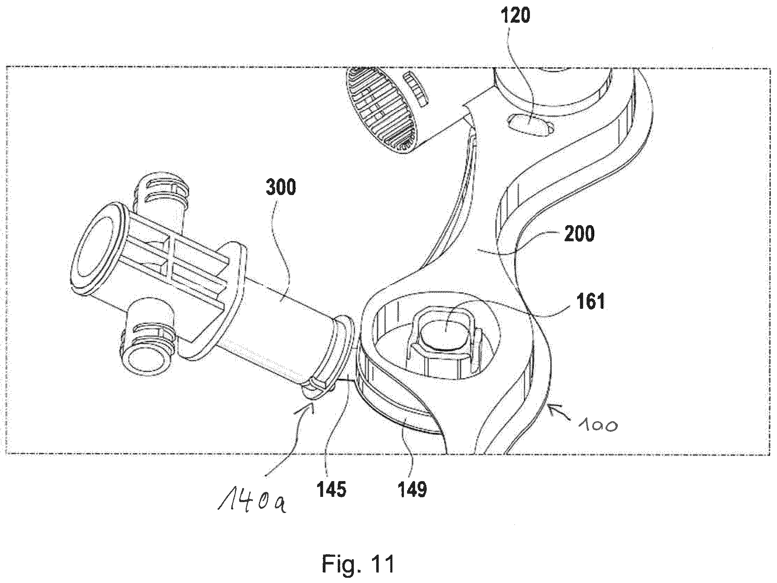

Als Beispiel für Schläuche, die mittels der Fixierungseinrichtung 140 gehalten werden können, seien hier Schläuche genannt, die in ihrem Gebrauch wenigstens einmal mittels eines Rinse-Port-Adapters 300 mit einem Rinse-Port der Fluidbehandlungsvorrichtung verbunden werden, der z. B. in Fig. 8 und Fig. 11 gezeigt ist.

Die Fixierungseinrichtung 140 ist im Beispiel der Fig. 7 als Halter für den Rinse-Port-Adapter 300 ausgestaltet, wobei vorgesehen ist, dass letzterer in ersten eingesteckt wird.

In Fig. 7 ist die Fixierungseinrichtung 140 mittels (hier optional) zweier Filmgelenke 141, 143 mit dem Hauptkörper der Berührschutzvorrichtung 100 verbunden. Die Filmgelenke 141, 143 liegen an gegenüberliegenden Enden eines, verglichen mit den Filmgelenken 141, 143 festeren oder steiferen, Abstandshalters 145. Sie erlauben ein Abbiegen der Fixierungseinrichtung 140 um 90° in eine Stellung, die in Fig. 8 zu sehen ist. Ersichtlich ist, dass es hierzu allerdings nicht mehr als eines Filmgelenks 141 oder 143 bedarf. Jedoch steigt mit zunehmender Anzahl an Filmgelenken die Flexibilität und Gelenkigkeit der Fixierungseinrichtung 140 an.

Im Beispiel der Fig. 7 sind Filmgelenke 141, 143 als Beispiele für Gelenke vorgesehen. Sie sind im Spritzguss gemeinsam mit dem Hauptkörper der Berührschutzvorrichtung 100 gefertigt, mit den bekannten Vorteilen. Ausführungsformen mit Gelenken, welche nicht gespritzt wurden, sind ebenso von der vorliegenden Erfindung umfasst.

Die konkrete Ausgestaltung der Filmgelenke 141, 143 erlaubt weiter vorteilhaft, auf Spritzformen ohne Schieber zurückgreifen zu können.

Die Fixierungseinrichtung 140 weist einen zylinderförmigen Einsteckabschnitt 147 auf, der von einer ringförmigen Struktur 149 umgeben ist. Zwischen dem zylinderförmigen Einsteckabschnitt 147 und der ringförmigen Struktur 149 liegt ein Spalt, in welchem das vordere Ende des Rinse-Port-Adapters 300, nach oder durch Einstecken und ggf. Verklemmen darin, gehalten werden kann.

**Fig. 8** zeigt die Berührschutzvorrichtung 100 der Fig. 7. Auf die Fixierungseinrichtung 140 ist ein Rinse-Port-Adapter 300 lösbar aufgesteckt.

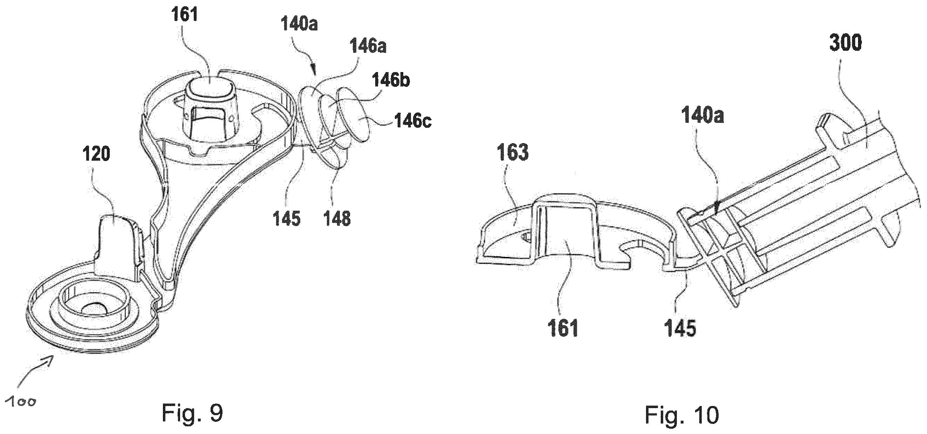

**Fig. 9** zeigt die Berührschutzvorrichtung 100 in Perspektive und von der Unterseite in einer dritten Ausführungsform mit einer Halte- oder Fixierungseinrichtung 140a. Abgesehen von der Fixierungseinrichtung 140a unterscheidet sich die Berührschutzvorrichtung 100 der Fig. 9 nicht von jener der vorangegangenen Figuren.

Die Ausführungsform der Fig. 9 kann ohne Schieber spritzgegossen werden. Es bedarf vorteilhafterweise zudem keines zu entkernenden Hinterschnitts.

Die Ausführungsform der Fig. 9 erlaubt eine flexible Anbindung zwischen Fixierungseinrichtung 140a und Hauptkörper der Berührschutzvorrichtung 100. Eine Biegung des flexiblen Halters um 45° ist in Fig. 9 gezeigt.

Die Fixierungseinrichtung 140a kann optional drei kreisförmige Strukturen oder Flächen 146a, 146b, 146c aufweisen. Aus Gründen der Zentrierung und besseren Verklemmung sind vorzugsweise wenigstens zwei von ihnen zum Eingriff in einen Hohlraum des Rinse-Port-Adapters 300 vorgesehen.

Optional sind bei der Berührschutzvorrichtung 100 in beliebiger Ausführungsform ein, zwei oder mehr elastische Sperrärmchen 148 vorgesehen, wie in Fig. 9 exemplarisch gezeigt. Sie können vorgesehen sein, um in die Nut in Umfangsrichtung einzuschnappen, die am Rinse-Port-Adapter 300 vorgesehen ist.

Auch nicht elastische Klemmabschnitte können ein lösbares Fixieren des Rinse-Port-Adapters 300 an der

Fixierungseinrichtung 140a alternativ oder ergänzend begünstigen.

Sowohl Klemmabschnitte als auch Sperrärmchen 148 können, wie in Fig. 9 exemplarisch gezeigt, in einer Längsrichtung des Rinse-Port-Adapters 300 stehen.

**Fig. 10** zeigt die Berührschutzvorrichtung 100 der Fig. 9, geschnitten sowohl durch den zweiten Verbindungsabschnitt 160 als auch durch die Halte- oder Fixierungseinrichtung 140a, mit leicht perspektivischem Blick auf die Schnittebene mit aufgesetztem Rinse-Port-Adapter 300 (ebenfalls im Schnitt).

**Fig. 11** zeigt die Berührschutzvorrichtung 100 der Fig. 9 und 10, verbunden mit dem Hartteil der Kassette 200. Der Rinse-Port-Adapter 300 ist auf die Fixierungseinrichtung 140a aufgesetzt.

Der Rinse-Port-Adapter 300 weist zwei integrale Luer-Konnektoren für die Patientenleitungen auf. Auf diese Weise kann die Halte- oder Fixierungseinrichtung 140a der Fig. 11 nicht nur die Patientenleitungen mittels der Berührschutzvorrichtung 100 kontaminationsfrei in der gewünschten Position an der Kassette 200 halten, sondern auch den aus dem Stand der Technik bekannten, separaten Deckel zum Verschließen des Rinse-Port-Adapters 300, einsparen helfen.

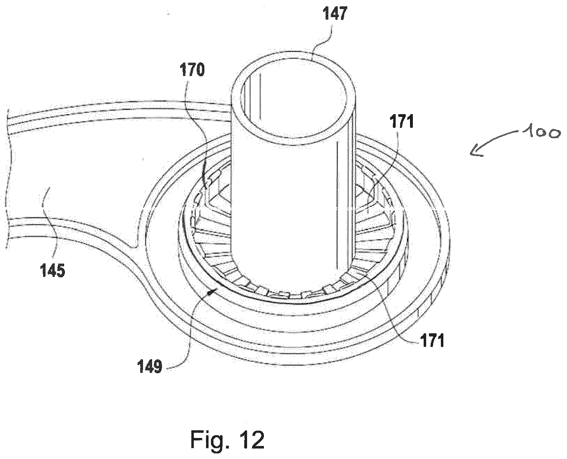

**Fig. 12** zeigt einen Teil der Berührschutzvorrichtung 100 in Perspektive und von der Unterseite in einer vierten Ausführungsform.

Anders als in den vorangegangenen Ausführungsformen weist die Berührschutzvorrichtung 100 eine Drainagestruktur 170 auf.

Die Drainagestruktur 170 weist hier exemplarisch Rillen oder Nuten 171 auf, oder besteht hieraus, welche sich z. B. radial von der in Fig. 12 nicht gezeigten Fixierungseinrichtung 140 (zu finden in vorangestellten Figuren), hier dem Einsteckabschnitt 147, erstrecken.

Im Beispiel der Fig. 12 erstrecken sich die Rillen oder Nuten 171 bodenseitig zwischen dem erhabenen Einsteckabschnitt 147 und der ringförmigen Struktur 149, die den Einsteckabschnitt 147 umgibt.

Die Rillen oder Nuten 171 erlauben vorteilhaft sterilem Dampf, oder einem anderen Sterilisiergas, während der Sterilisierung der Kassette 200 mit aufgesetzter Berührschutzvorrichtung 100 an verschiedene - wenn nicht alle - Flächen des Rinse-Port-Adapters 300 zu gelangen, selbst wenn dieser auf den Einsteckabschnitt 147 aufgesteckt ist.

### Bezugszeichenliste

- 100: Berührschutzvorrichtung
- 110: Abdeckabschnitt
- 111: erste Struktur
- 113: zweite Struktur
- 120: erster Verbindungsabschnitt
- 121: Abstandshalter
- 130: Kodierstruktur
- 140: Fixierungseinrichtung
- 140a: Fixierungseinrichtung
- 141: Filmgelenk
- 143: Filmgelenk
- 145: Abstandshalter
- 146a: kreisförmige Fläche
- 146b: kreisförmige Fläche
- 146c: kreisförmige Fläche
- 147: Einsteckabschnitt
- 148: elastische Sperrärmchen
- 149: ringförmige Struktur
- 150: Griffabschnitt
- 151: Struktur
- 151a: Vertiefung
- 151b: Vertiefung
- 160: zweiter Verbindungsabschnitt
- 161: Erhebung
- 161a: Vorsprung
- 161b: Vorsprung
- 163: Struktur
- 163a: Vertiefung
- 163b: Vertiefung
- 165: erste Durchgangsöffnung
- 167: zweite Durchgangsöffnung
- 170: Drainagestruktur
- 171: Rillen oder Nuten

- 200: fluidführende Kassette
- 241: Konnektionsstelle
- 241a: Kanalstruktur
- 242: Zentrieröffnung oder Halteöffnung
- 251: Steg
- 252: umlaufende Struktur
- 253: Konnektor
- 255: Konnektor
- 261a: Nase
- 261b: Nase
- 261c: Nase
- 265: Erhebung
- 267: Erhebung

- 300: Rinse-Port-Adapter

## Patentansprüche

1. Medizinische fluidführende Kassette (200) für eine medizinische Fluidbehandlung, mit einer Konnektionsstelle (241), einer Zentrieröffnung (242) und wenigstens einer Berührschutzvorrichtung (100) zum Abdecken der Konnektionsstelle (241), wobei die Berührschutzvorrichtung (100) eine Oberseite und eine Unterseite mit wenigstens einem Abdeckabschnitt (110) zum Abdecken der Konnektionsstelle (241) vor Gebrauch der Kassette (200) aufweist, und wobei die Berührschutzvorrichtung (100) wenigstens einen ersten (120) und einen zweiten (160) Verbindungsabschnitt zum lösbaren Verbinden der Berührschutzvorrichtung (100) mit der Kassette (200) oder zum lösbaren Halten der Berührschutzvorrichtung (100) an der Kassette (200) umfasst, wobei der erste Verbindungsabschnitt eine Einsteck- oder Rastverbindung oder Teil hiervon ist und wobei der zweite Verbindungsabschnitt (160) eine Erhebung (161) zum Einführen in die Zentrieröffnung (242) der fluidführenden Kassette (200) aufweist.

2. Kassette (200) nach Anspruch 1, wobei der Abdeckabschnitt (110) wenigstens eine erste Struktur (111) aufweist, welche in ihrem Umfang geschlossen ist.

3. Kassette (200) nach Anspruch 1 oder 2, wobei der Abdeckabschnitt (110) wenigstens eine zweite Struktur (113) aufweist, welche in ihrem Umfang geschlossen ist.

4. Kassette (200) nach einem der vorangegangenen Ansprüche, wobei die Berührschutzvorrichtung (100) wenigstens einen Griffabschnitt (150) aufweist, und wobei der Griffabschnitt (150) wenigstens eine dritte Struktur (151) aufweist, welche in ihrem Umfang geschlossen ist.

5. Kassette (200) nach Anspruch 4, wobei die dritte Struktur (151) wenigstens eine Vertiefung (151a, 151b) in ihrem äußersten Rand aufweist.

6. Kassette (200) nach einem der vorangegangenen Ansprüche, wobei der zweite Verbindungsabschnitt (160) eine vierte Struktur (163) aufweist, welche in ihrem Umfang geschlossen ist.

7. Kassette (200) nach Anspruch 6, wobei die vierte Struktur (163) wenigstens eine Vertiefung (163a, 163b) in ihrem äußersten Rand aufweist.

8. Kassette (200) nach einem der vorangegangenen Ansprüche, wobei der Griffabschnitt (150) zwischen dem ersten Verbindungsabschnitt (120) und dem zweiten Verbindungsabschnitt (160) angeordnet ist.

9. Kassette (200) nach einem der vorangegangenen Ansprüche, wobei die Berührschutzvorrichtung (100) im Bereich des zweiten Verbindungsabschnitts (160) wenigstens eine erste Durchgangsöffnung (165) aufweist.

10. Kassette (200) nach einem der vorangegangenen Ansprüche, wobei die Berührschutzvorrichtung (100) im Bereich des ersten Verbindungsabschnitts (120) wenigstens einen Abstandshalter (121) aufweist.

11. Kassette (200) nach einem der vorangegangenen Ansprüche, wobei die Berührschutzvorrichtung (100) wenigstens eine Kodierstruktur (130) zum formbedingt kodierten Anordnen der Berührschutzvorrichtung (100) an der fluidführenden Kassette (200) aufweist.

12. Kassette (200) nach einem der vorangegangenen Ansprüche, wobei die Berührschutzvorrichtung (100) einteilig hergestellt ist.

13. Kassette (200) nach einem der vorangegangenen Ansprüche, wobei der Verbindungsabschnitt (120) der Berührschutzvorrichtung (100) diese mit einer Halteöffnung oder der Zentrieröffnung (242) der Kassette (200) für deren Aufnahme an einer Blutbehandlungsvorrichtung verbindet.

14. Kassette (200) nach einem der vorangegangenen Ansprüche, wobei die Konnektionsstelle (241) eine Verbindungsstelle für eine Substituatleitung ist.

15. Kassette (200) nach einem der vorangegangenen Ansprüche, wobei die Tiefe der Zentrieröffnung (242) der fluidführenden Kassette (200) größer ist als die Höhe der Erhebung (161) des zweiten Verbindungsabschnitts (160).

## Claims

1. A medical fluid-guiding cassette (200) for a medical fluid treatment, comprising a connection point (241), a centering opening (242), and at least a contact protection device (100) for covering the connection point (241), wherein the contact protection device (100) comprises a top side and a bottom side with at least one cover section (110) for covering the connection point (241) before use of the cassette (200), and wherein the contact protection device (100) comprises at least a first (120) and a second (160) connecting portion for releasably connecting the contact protection device (100) to the cassette (200) or for releasably holding the contact protection device (100) at the cassette (200), wherein the first connecting section is a plug-in or snap-in connection or part thereof, and wherein the second connecting section (160) comprises a projection (161) for insertion into the centering opening (242) of the fluid-carrying cassette (200).

2. The cassette (200) according to claim 1, wherein the cover section (110) comprises at least one first structure (111) which is closed in its circumference.

3. The cassette (200) according to claim 1 or 2, wherein the cover section (110) comprises at least one second structure (113) which is closed in its circumference.

4. The cassette (200) according to any one of the preceding claims, wherein the contact protection device (100) comprises at least one handle portion (150), and wherein the handle portion (150) comprises at least a third structure (151) which is closed in its circumference.

5. The cassette (200) according to claim 4, wherein the third structure (151) comprises at least one indentation (151a, 151b) in its outermost rim.

6. The cassette (200) according to any one of the preceding claims, wherein the second connecting portion (160) comprises a fourth structure (163) which is closed in its circumference.

7. The cassette (200) according to claim 6, wherein the fourth structure (163) comprises at least one indentation (163a, 163b) in its outermost rim.

8. The cassette (200) according to any one of the preceding claims, wherein the handle portion (150) is arranged between the first connecting portion (120) and the second connecting portion (160).

9. The cassette (200) according to any one of the preceding claims, wherein the contact protection device (100) comprises at least one first through-opening (165) in the region of the second connecting portion (160).

10. The cassette (200) according to any one of the preceding claims, wherein the contact protection device (100) comprises at least one spacer (121) in the region of the first connecting portion (120).

11. The cassette (200) according to any one of the preceding claims, wherein the contact protection device (100) comprises at least one coding structure (130) for the shape-induced coded arrangement of the contact protection device (100) at the fluid-guiding cassette (200).

12. The cassette (200) according to any one of the preceding claims, wherein the contact protection device (100) is integrally formed.

13. The cassette (200) according to any one of the preceding claims, wherein the connecting portion (120) of the contact protection device (100) connects the latter to a retaining opening or to the centering opening (242) of the cassette (200) for its reception on a blood treatment apparatus.

14. The cassette (200) according to any one of the preceding claims, wherein the connection point (241) is a connection point for a substitute line.

15. The cassette (200) according to any one of the preceding claims, wherein the depth of the centering opening (242) of the fluid-carrying cassette (200) is greater than the height of the projection (161) of the second connecting portion (160).

## Revendications

1. Une cassette conductrice de fluide médical (200) pour un traitement médical par fluide, comprenant un point de raccordement (241), une ouverture de centrage (242) et au moins un dispositif de protection contre les contacts (100) destiné à recouvrir le point de raccordement (241), où le dispositif de protection contre les contacts (100) présente une face supérieure et une face inférieure avec au moins une section de recouvrement (110) destinée à recouvrir le point de raccordement (241) avant utilisation de la cassette (200), et où le dispositif de protection contre les contacts (100) comprend au moins une première (120) et une seconde (160) section de raccordement permettant de raccorder de manière amovible le dispositif de protection contre les contacts (100) à la cassette (200) ou de maintenir de manière amovible le dispositif de protection contre les contacts (100) avec la cassette (200),
où la première section de raccordement est un raccordement enfichable ou encliquetable, ou une partie de celui-ci, et où la seconde section de raccordement (160) présente une saillie (161) destinée à être insérée dans l'ouverture de centrage (242) de la cassette conductrice de fluide (200).

2. La cassette (200) selon la première revendication, où la section de recouvrement (110) présente au moins une première structure (111) qui est fermée dans sa circonférence.

3. La cassette (200) selon la revendication 1 ou 2, où la section de recouvrement (110) présente au moins une seconde structure (111) qui est fermée dans sa circonférence.

4. La cassette (200) selon l'une quelconque des revendications précédentes, où le dispositif de protection contre les contacts (100) présente au moins une partie de préhension (150), et où la partie de préhension (150) comprend au moins une troisième structure (151) qui est fermée dans sa circonférence.

5. La cassette (200) selon la revendication 4, où la troisième structure (151) comprend au moins une indentation (151a, 151b) sur son rebord le plus extérieur.

6. La cassette (200) selon l'une quelconque des revendications précédentes, où la seconde section de raccordement (160) comprend une quatrième structure (163) qui est fermée dans sa circonférence.

7. La cassette (200) selon la revendication 6, où la quatrième structure (163) comprend au moins une indentation (163a, 163b) sur son rebord le plus extérieur.

8. La cassette (200) selon l'une quelconque des revendications précédentes, où la partie de préhension (150) est agencée entre la première section de raccordement (120) et la seconde section de raccordement (160).

9. La cassette (200) selon l'une quelconque des revendications précédentes, où le dispositif de protection contre les contacts (100) comprend au moins une première ouverture de passage (165) dans la zone de la seconde section de raccordement (160).

10. La cassette (200) selon l'une quelconque des revendications précédentes, où le dispositif de protection contre les contacts (100) comprend au moins une entretoise (121) dans la zone de la première section de raccordement (160).

11. La cassette (200) selon l'une quelconque des revendications précédentes, où le dispositif de protection contre les contacts (100) comprend au moins une structure de codage (130) pour l'agencement codé induit par la forme du dispositif de protection contre les contacts (100) avec la cassette conductrice de fluide (200).

12. La cassette (200) selon l'une quelconque des revendications précédentes, où le dispositif de protection contre les contacts (100) est réalisé d'une seule pièce.

13. La cassette (200) selon l'une quelconque des revendications précédentes, où la section de raccordement (120) du dispositif de protection contre les contacts (100) relie ce dernier à une ouverture de retenue ou à l'ouverture de centrage (242) de la cassette (200) pour son logement sur un appareil de traitement du sang.

14. La cassette (200) selon l'une quelconque des revendications précédentes, où le point de raccordement (241) est un point de raccordement pour un conduit de substitution.

15. La cassette (200) selon l'une quelconque des revendications précédentes, où la profondeur de l'ouverture de centrage (242) de la cassette conductrice de fluide (200) est supérieure à la hauteur de la saillie (161) de la seconde section de raccordement (160).
